# EUROPEAN PATENT SPECIFICATION

(11) **EP 1 172 075 B1**
(45) Date of publication and mention of the grant of the patent: **15.09.2004**
(21) Application number: 00114765.1
(22) Date of filing: 10.07.2000
(51) Int. Cl.: A61F 5/01

(54) **Device for preventing hand contraction, particularly during sleep**
Vorrichtung zur Verhinderung der Kontraktion der Hand, insbesondere während des Schlafes
Dispositif prévenant la contraction de la main, en particulier pendant le sommeil

(43) Date of publication of application: 16.01.2002
(73) Proprietor: Daglio, Anna Maria, 15067 Novi Ligure (Alessandria) (IT)
(72) Inventor: Daglio, Anna Maria, 15067 Novi Ligure (Alessandria) (IT)
(74) Representative: Giambrocono, Alfonso, Dr. Ing.

(56) References cited:
- US-A- 5 069 203
- US-A- 5 807 293
- US-A- 5 921 945

## Description

This invention relates to a device for preventing hand contraction, particularly sleep.

It is well known that many people are unable to relax completely and remain in a state of psychophysical tension even during sleep, resulting in insufficient rest during the night, with awakening associated with a sensation of tiredness.

This state is normally associated with incomplete muscular relaxation, in which the limbs remain contracted, the hands clutched and the jaws clenched, this latter phenomenon being known as bruxism.

These phenomena are normally considered to relate to the structuration of a reaction, which is initially occasional but then becomes consolidated in time with the persistence of the basic cause.

At this point a circular flow of information and impulses is established which starting from the motory area of the brain maintains the hand muscles in a tonic condition of alertness, a phenomenon not dissimilar to that which sustains contracture of the neck and shoulder muscles and the nocturnal contraction of the jaw muscles.

As in the case of bruxism, muscular contracture also persists for the hands during nocturnal rest, to often constitute an obstacle to regenerative sleep.

In this respect the continuous flow of motory stress hinders the generation of that state of complete relaxation which is essential for sleep to become an optimum factor in physical and mental recuperation.

The sleep of a bruxism subject and of a subject sleeping with the hands contracted (these two conditions being nearly always associated with each other) is superficial and often interrupted, and on awakening results in muscular stiffness and difficulty in physical and mental revival.

This condition can occur with various levels of intensity, all to be taken into careful consideration to prevent slow but inevitable continuous psychophysical wear and tear.

It has been demonstrated that a complete state of neuro-muscular relaxation cannot exist in the body if even one of its sectors is in a state of tension, and that, in contrast, by completely relaxing even a single limited sector of its musculature the other musculature sectors are also relaxed, together with all sectors of mental activity.

The object of this invention is to provide a device able to prevent contraction of the hand, in order to facilitate complete relaxation of all other muscles.

In this respect, it has been found that by preventing contraction of the flexor muscles, relaxation not only of the hand but also of the other habitually contracted muscles is obtained in a relatively short period, to induce in the patient new habits which then persist with time.

US 5 921 945 discloses a splint device to aid in the healing and rehabilitation process after injury, surgery or the like, which is able to prevent hand contraction. This device has a substantially elastomeric element for being applied to the palm of the hand and involves the fingers, and comprises straps associated with said element to prevent flexure of the hand.

To attain this object, the invention provides a device for obtaining complete muscular relaxation during the sleep, according to claim 1.

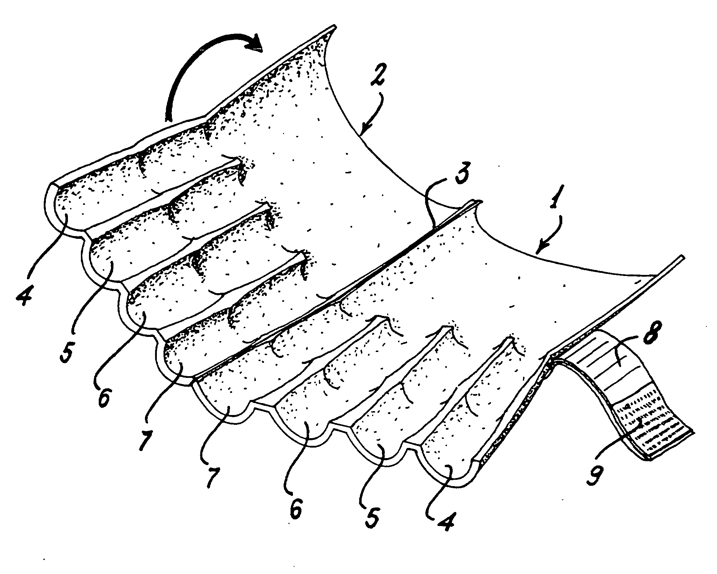

The invention will be more apparent from the detailed description of one embodiment thereof given hereinafter by way of non-limiting example with reference to the accompanying drawing, which shows a perspective view of one implementation of the inventive concept on which the invention is based.

In said figure the reference numerals 1 and 2 indicate two half-casings, one palmar and the other dorsal, which are joined together by a hinge region 3 and comprise extensions 4, 5, 6, 7 arranged to receive respectively the index, middle, ring and little finger of the hand.

If, as is convenient, the device is constructed of a suitable plastic material, the hinge region can be formed by simply moulding the two half-casings so that they are connected together at 3, and providing weakened portions forming the preferential hinging axis. Alternatively, protuberances can be provided on one half-casing to snap-fit into suitable seats provided in the other half-casing.

The operation of the device is intuitive: the two half-casings are opened out, the palm of the hand is positioned on the palmar half-casing, the second half-casing is closed thereonto and is secured by any closure means. In the illustrated example, a strap 8 fixed to one half-casing has a portion provided with "velcro" 9, which clamps onto the other half-casing.

Normally, to make the device more comfortable, it can be lined internally with a suitable fabric.

In a modified embodiment, the two half-casings can be separate from each other and be connected together by a number of straps on one and the other side.

Numerous other variants are possible, for example the dorsal half-casing can be replaced by an elasticized band, or the dorsal half-casing alone can be provided and be retained in situ by wearing over it a normal glove, or a glove can be used having its fingers and palm stiffened in the palmar part, for example by sticks involving at least part of the fingers and palm, these variants all falling within the scope of the invention.

In a preferred embodiment, the device is formed in such a manner as to maintain the hand perfectly outstretched and the fingers slightly divergent.

The extended position of the fingers which hence arises results initially in some slight forcing to overcome both the unusual habit of diverging the fingers and the now habitual tension of the flexor muscles of the hand. A certain time is therefore required to achieve complete relaxation of the hand muscles, this having suggested the concept of using the device for part or all of the nocturnal rest period.

The initial difficulty encountered in maintaining the unusual position of the fingers and palm of the hand is easily overcome within the first few applications, especially as the benefits deriving from increased sleep profondity become apparent.

All subjects tested have immediately encountered less agitated sleep, more ready awakening and increased energy in resuming their normal occupations.

The tests carried out with the device of the invention have shown a clear improvement in induced relaxation compared with other empirical methods used up to the present time, particularly, as is preferable, the device is used during sleep.

## Claims

1. Device for preventing hand contraction during the sleep, comprising an element to be applied to the hands, said element being associated with means acting on the hand to retain said element in situ said device involving each finger except the thumb, **characterised in that** said element comprises two half-casings (1, 2), one palmar and the other dorsal, at least one of those being rigid, and **in that** the element involves only the first two phalanges of each finger and has means (8) for coupling the two half-casings.

2. Device as claimed in claim 1, **characterised by** the fact that both said half-casings are rigid.

3. Device as claimed in claims 1 and 2, **characterised by** the fact that said means acting to retain said element in situ consists in means (8, 9) for coupling the two half-casings.

4. Device as claimed in claim 3, **characterised by** the fact that said means (8, 9) are elastic.

5. Device as claimed in claims 1 and 2, **characterised by** the fact that said means acting to retain said element in situ consists of gloves to be worn over the palmar half-casing.

## Patentansprüche

1. Vorrichtung zum Vermeiden von Handkontraktion während des Schlafes, umfassend ein an die Hände anzubringendes Teil, wobei das Teil mit einem Mittel verknüpft ist, welches auf die Hand wirkt, um das Teil an Ort und Stelle zu halten, wobei die Vorrichtung jeden Finger außer den Daumen einbezieht,
**dadurch gekennzeichnet,**
**dass** das Teil zwei Halbgehäuse (1, 2) umfasst, eins palmar und das andere dorsal, von welchen zumindest eins steif ist, und dadurch, dass das Teil nur die ersten zwei Fingerglieder jedes Fingers einbezieht und Mittel (8) zum Koppeln der zwei Halbgehäuse aufweist.

2. Vorrichtung wie in Anspruch 1 beansprucht, **gekennzeichnet durch** die Tatsache, dass beide der Halbgehäuse steif sind.

3. Vorrichtung wie in Anspruch 1 und 2 beansprucht, **gekennzeichnet durch** die Tatsache, dass das Mittel, welches dazu dient, das Teil an Ort und Stelle zu halten, aus Mitteln (8, 9) zum Koppeln der zwei Halbgehäuse besteht.

4. Vorrichtung wie in Anspruch 3 beansprucht, **gekennzeichnet durch** die Tatsache, dass die Mittel (8, 9) elastisch sind.

5. Vorrichtung wie in Anspruch 1 und 2 beansprucht, **gekennzeichnet durch** die Tatsache, dass das Mittel, welches dazu dient, das Teil an Ort und Stelle zu halten, aus Handschuhen besteht, welche über den palmaren Halbgehäuse zu tragen sind.

## Revendications

1. Dispositif destiné à empêcher la contraction de la main lors du sommeil, comportant un élément devant être appliqué aux mains, ledit élément étant associé à un moyen agissant sur la main pour retenir ledit élément in situ, ledit dispositif concernant chaque doigt sauf le pouce, **caractérisé en ce que** ledit élément comporte deux demi-gaines (1, 2), l'une palmaire et l'autre dorsale, au moins une de celles-ci étant rigide, et **en ce que** l'élément ne concerne que les deux premières phalanges de chaque doigt et a un moyen (8) destiné à coupler les deux demi-gaines.

2. Dispositif tel que revendiqué dans la revendication 1, **caractérisé par le fait que** les deux demi-gaines sont rigides.

3. Dispositif tel que revendiqué dans les revendications 1 et 2, **caractérisé par le fait que** ledit moyen agissant pour retenir ledit élément in situ est composé de moyens (8, 9) destinés à coupler les deux demi-gaines.

4. Dispositif tel que revendiqué dans la revendication 3, **caractérisé par le fait que** lesdits moyens (8, 9) sont élastiques.

5. Dispositif tel que revendiqué dans les revendications 1 et 2, **caractérisé par le fait que** ledit moyen agissant pour retenir ledit élément in situ consiste en des gants devant être portés pardessus la demi-gaine palmaire.
